# EUROPÄISCHE PATENTANMELDUNG

(11) **EP 3 882 335 A1**
(43) Veröffentlichungstag der Anmeldung: **22.09.2021**
(21) Anmeldenummer: 21158569.0
(22) Anmeldetag: 23.02.2021
(51) Int. Cl.: C12M 3/06, C12M 1/00, C12M 3/00, C12M 1/12, C12M 1/34

(54) **VERFAHREN ZUR KULTIVIERUNG VON ZELLEN**

(30) Priorität: 19.03.2020 DE 102020107599
(71) Anmelder: ibidi GmbH, 82166 Gräfelfing (DE)
(72) Erfinder: von Guttenberg, Zeno, 82166 Gräfelfing (DE); Hagmeyer, Britta, 72770 Reutlingen (DE); Werner, Simon, 72770 Reutlingen (DE); Schmees, Christian, 72770 Reutlingen (DE); Pawlak, Michael, 72770 Reutlingen (DE); Stelzle, Martin, 72770 Reutlingen (DE)
(74) Vertreter: Grünecker Patent- und Rechtsanwälte PartG mbB

(57) **Zusammenfassung**

Die Erfindung betrifft ein Verfahren zur Kultivierung von Zellen in einem Substrat, in dem eine Kammer mit mindestens einer Seitenwand, einem Boden, und einer Decke ausgebildet ist, umfassend Einbringen von Zellen in die Kammer, Kippen des Substrats derart, dass die Zellen sich an einer Seitenwand der Kammer ansammeln, und Halten des Substrats in der gekippten Ausrichtung, so dass die Zellen ein dreidimensionales Zellaggregat bilden.

## Beschreibung

Die Erfindung betrifft ein Verfahren zur Kultivierung von Zellen. Das Verfahren umfasst das Bilden eines dreidimensionalen Zellaggregats.

In dreidimensionalen Zellaggregaten, beispielsweise Zellsphäroiden, haften einzelne Zellen aneinander und stellen damit einen Zellverband dar, der eher den physiologischen Verhältnissen (Organstrukturen) bei Menschen und Tieren entspricht als dies für eine zweidimensionale Zellkultur in einem Monolayer der Fall ist.

Obwohl eine zweidimensionale Zellkultur weniger gut für Wirkstofftests geeignet ist als ein dreidimensionales Zellaggregat, wird diese häufiger verwendet. Das liegt beispielsweise an der schlechteren Handhabbarkeit bei der Herstellung und Untersuchung von dreidimensionalen Zellaggregaten.

Typische Zellkultur-Verfahren umfassen, dass Zellaggregate gebildet werden, anschließend perfundiert und während und/oder nach der Perfusion untersucht werden.

Viele bekannte Systeme benötigen dafür mindestens zwei verschiedene Plattformen oder Substrate, die nacheinander verwendet werden. Beispielsweise erfolgt bei manchen dieser Systeme eine Assemblierung von Einzelzellen zu einem Zellaggregat, indem die einzelnen Zellen zunächst in speziell dafür geeignete Kulturplatten pipettiert werden. Diese können beispielsweise Trichterstrukturen oder Töpfchen mit rundem Boden enthalten, die mit einer zellabweisenden Beschichtung modifiziert sind, um eine Adhäsion an den Oberflächen zu verhindern. Statt einer Adhäsion an der Oberfläche erfolgt dann ein Aufbau von Zellaggregaten, deren Form der Töpfchenform entspricht. Alternativ werden Platten zur Erzeugung von hängenden Tropfen verwendet, wobei die Tropfen jeweils als antiadhäsives Zellgefäß dienen. Alternativ können Zellaggregate in Haltestrukturen in einem Fluidkanal assembliert werden, nämlich durch Wassertröpfchen in Öl. In den Wassertröpfchen wird dazu eine Polymermatrix erzeugt, in die sich Zellen einlagern können. Das Öl wird später wieder weggespült. Anschließend werden die assemblierten Zellaggregate in separate Perfusions- und/oder Untersuchungsbereiche übertragen bzw. transportiert, die eine separate Perfusion und/oder Untersuchung der Zellaggregate ermöglichen.

Eine solche Übertragung bzw. ein solcher Transport ist aufwändig und kann die Qualität der Zellaggregate negativ beeinflussen. Solche Verfahren sind zudem hinsichtlich der Form der Zellkultur sehr eingeschränkt, weil die mögliche Form der Zellaggregate auf Tropfen- oder Töpfchenform beschränkt ist.

Andere Systeme ermöglichen zwar, solch eine Übertragung bzw. einen Transport der Zellaggregate zu vermeiden, wie beispielsweise in der US 2016/097028 A1 beschrieben. In diesem System wird ein mikrofluidischer Kanal mit quadratischen Vertiefungen am Kanalboden, in denen sich dreidimensionale Zellkulturen bilden, verwendet. Die Form der Zellkulturen entspricht im Wesentlichen der Form der Vertiefung. Die Zellkulturen können perfundiert und mittels Mikroskopie untersucht werden. Der Nachteil dieses System ist, dass wenig Flexibilität hinsichtlich der Form der Vertiefungen besteht, wenn die Zellkulturen eine bestimmte Form und Größe annehmen sollen. Demnach können die Vertiefungen nur begrenzt an Anforderungen für die Perfusion, beispielsweise hinsichtlich des Strömungsverhaltens, oder an Anforderungen für optische Untersuchungen angepasst werden.

Es ist eine Aufgabe der Erfindung ein Verfahren, ein Substrat und ein System zur Kultivierung von Zellen bereitzustellen, die ermöglichen, auf einfache Weise dreidimensionale Zellaggregate zu erhalten, mit geringen Einschränkungen hinsichtlich der Form des Perfusions- und Untersuchungsbereichs.

Das erfindungsgemäße Verfahren zur Kultivierung von Zellen in einem Substrat, in dem eine Kammer mit mindestens einer Seitenwand, einem, insbesondere planen, Boden, und einer Decke ausgebildet ist, umfasst das Einbringen von Zellen in die Kammer, Kippen des Substrats in eine gekippte Ausrichtung derart, dass die Zellen sich an einer Seitenwand der Kammer ansammeln, Halten des Substrats in der gekippten Ausrichtung, so dass die Zellen ein dreidimensionales Zellaggregat bilden.

Das Verfahren ist einfach und wenig aufwändig und verschiedene Formen von Zellaggregaten können einfach hergestellt werden, was unten noch im Detail erläutert wird. So ist auch die Flexibilität hinsichtlich der Aggregatsform hoch. Die Form, insbesondere auch Bodenform, und die Größe der Kammer sind nicht weiter beschränkt, so dass auch dadurch hohe Flexibilität hinsichtlich optischer Untersuchungen und Perfusion möglich ist. Insbesondere ermöglicht das Verfahren, die Bildung eines Zellaggregats und die anschließende Perfusion und Untersuchung in derselben Kammer durchführen zu können, so dass auf umfangreiche Übertragungsschritte verzichtet werden kann.

Das Ansammeln an einer Seitenwand kann umfassen, dass sich Zellen an nur einer einzigen Seitenwand oder an einer Kante zwischen zwei Seitenwänden ansammeln. Dies kann durch die Kipprichtung beeinflusst werden.

Bei der Kammer kann es sich um eine Kultivierungs- und/oder Beobachtungskammer handeln, insbesondere eine Beobachtungskammer für Mikroskopie-Untersuchungen. Die Kammer kann als ein in dem Substrat ausgebildeter Hohlraum betrachtet werden, der durch den Boden und die Seitenwand bzw. Seitenwände und den Deckel begrenzt ist, die jeweils durch das Substrat gebildet werden.

Das Bilden eines Zellaggregats wird auch als "Assemblieren" bezeichnet. Dass sich ein Zellaggregat bildet, umfasst, dass die Zellen aneinanderwachsen. Die Bildung des Zellaggregats kann insbesondere umfassen, dass die Zellen etwa in der Formation zusammenwachsen, in der sie sich angesammelt haben.

Das Zellaggregat kann beispielsweise kugelförmig sein. Beispielhaft kann das kugelförmige Zellaggregat etwa einen Durchmesser von 100 bis 650, insbesondere 150 bis 600, insbesondere 200 bis 550, insbesondere 250 bis 500 Mikrometer aufweisen. Das kugelförmige Zellaggregat kann beispielsweise 3000 bis 7000, insbesondere 4000 bis 6000, insbesondere 4500 bis 5500 Zellen, insbesondere etwa 5000 Zellen aufweisen. Insbesondere kann das Zellaggregat kugelförmig sein, einen Durchmesser von 250 bis 500 Mikrometer aufweisen und 4500 bis 5500, insbesondere etwa 5000 Zellen aufweisen.

Das Zellaggregat kann alternativ beispielsweise stäbchenförmig sein. Das stäbchenförmige Zellaggregat kann etwa einen Durchmesser von 100 bis 650, insbesondere 150 bis 600, insbesondere 200 bis 550, insbesondere 250 bis 500 Mikrometer aufweisen, und eine Länge, die größer ist als der Durchmesser. Insbesondere kann das stäbchenförmige Zellaggregat eine Länge von 700 Mikrometer bis 5 mm, insbesondere 1 mm bis 4,8 mm, insbesondere eine Länge von 1,5 mm bis 4,6 mm, insbesondere 2 mm bis 4,4 mm, insbesondere 2,5 mm bis 4,2 mm, insbesondere 3 mm bis 4,1 mm, insbesondere 3,5 mm bis 4 mm, insbesondere etwa 4 mm, aufweisen. Das stäbchenförmige Zellaggregat kann beispielsweisen zwischen 3000 und 45000 Zellen aufweisen, insbesondere zwischen 5000 und 40000. Das stäbchenförmige Zellaggregat kann Insbesondere kann das Zellaggregat zwischen 3,5 und 4 mm lang sein, einen Durchmesser von 250 und 500 Mikrometer aufweisen und zwischen 35000 und 40000 Zellen enthalten.

Das Verfahren zur Kultivierung von Zellen in einem dreidimensionalen Zellaggregat kann das Assemblieren und Kultivieren einer dreidimensionalen Zellkultur umfassen. Solche Verfahren werden auch als 3D-Zellkultur bezeichnet. Es handelt sich hier insbesondere um eine sogenannte dynamische 3D-Zellkultur, bei der das Assemblieren und Perfundieren in einer Plattform kombiniert werden.

Das Substrat kann eine Fluidstruktur, insbesondere eine Mikrofluidstruktur aufweisen. Es kann als mikrofluidische Plattform betrachtet werden. Bei dem Substrat kann es sich insbesondere um eines der unten beschriebenen Substrate handeln. Insbesondere kann die Kammer so ausgebildet sein, wie unten im Zusammenhang mit den erfindungsgemäßen Substraten beschrieben.

Bei den Zellen kann es sich beispielsweise um Tumorzellen oder Zellen, die am Aufbau von Organen, Knochen, Sehnen und Knorpel beteiligt sind, handeln.

Das Einbringen der Zellen in die Kammer kann umfassen, dass eine Zellsuspension durch einen Fluidanschluss in das Substrat eingeleitet wird und durch einen Fluidkanal in die Kammer transportiert wird, beispielsweise mittels einer Pumpe oder durch Kippen des Substrats. Alternativ kann das Einbringen auch ein Pipettieren unmittelbar in die Kammer umfassen. Der Fluidanschluss kann auf der Oberseite des Substrats angeordnet sein. Bei den Fluidanschlüssen kann es sich um Luer-Anschlüsse handeln.

Das Kippen des Substrats derart, dass die Zellen sich an einer Seitenwand der Kammer ansammeln, kann ein Kippen um die Längsachse oder die Querachse des Substrats oder um eine andere zur Hochachse senkrechte Achse, beispielsweise die Diagonale, erfolgen. Die Achse richtet sich danach, in welchem Bereich der Kammer das Zellaggregat gebildet werden soll. Das Kippen kann beispielsweise bewirken, dass sich die Zellen gravitationsbedingt an die Seitenwand bzw. Seitenwände bewegen, beispielsweise rutschen.

Die Drehachse und Richtung, in die das Substrat gekippt wird, können insbesondere jeweils eine Drehachse und Richtung sein, die dazu führt, dass durch das Kippen die Seitenwand bzw. Seitenwände der Kammer, an der bzw. denen sich die Zellen ansammeln relativ zum Rest der Kammer weiter unten angeordnet wird.

Wie lange das Substrat mindestens in der gekippten Ausrichtung gehalten wird, hängt davon ab, um welchen Zelltyp es sich handelt und wie viele Zellen das Zellaggregat bilden. Die Dauer, die das Zellaggregat in der gekippten Ausrichtung gehalten wird, kann entweder groß genug gewählt werden, dass für alle möglichen Szenarien auf jeden Fall ein Zellaggregat gebildet wird. Alternativ kann die Mindestdauer für den jeweiligen Fall empirisch oder semi-empirisch ermittelt werden.

Das Verfahren kann ein Zurückkippen des im Anschluss an das Bilden des Zellaggregats umfassen. Das Zurückkippen des Substrats kann umfassen, dass das Substrat wieder in die Ausgangsposition, also die Position vor dem Kippen gebracht wird. Zusätzlich dazu kann das Substrat optional durch die Ausgangsposition hindurch gekippt und anschließend wieder in die Ausgangsposition gebracht werden.

Das Verfahren kann eine Perfusion des Zellaggregats in der Kammer umfassen, insbesondere während des Zurückkippens und/oder nach dem Zurückkippen, wobei das Zurückkippen insbesondere ein Zurückkippen in eine Ausgangsposition, aus der es in die gekippte Ausrichtung gekippt wurde, umfasst.

Perfusion bedeutet, dass Flüssigkeit, insbesondere Zellkulturmedium, an dem Zellaggregat vorbei strömt oder dieses umströmt. Wenn der verwendete Zelltyp erfordert, dass die Nährstoffkonzentration im umgebenden Zellmedium konstant hoch ist, kann beispielsweise ein Umströmen durchgeführt werden. Wenn der verwendete Zelltyp empfindlich gegenüber Scherströmungen, kann beispielsweise Superperfusion verwendet werden.

Das Substrat kann mehrere Kammern umfassen und vor dem Kippen können in jede der Kammern Zellen eingebracht werden, so dass durch das Kippen und Halten des Substrats gleichzeitig in jeder der Kammern ein Zellaggregat gebildet wird. Das Verfahren kann dann eine Perfusion eines oder mehrerer der, insbesondere aller, Zellaggregate, insbesondere unabhängig voneinander, umfassen. Insbesondere kann das Verfahren eine gleichzeitige Perfusion mehrerer, insbesondere aller, Zellaggregate umfassen.

Der Vorteil ist, dass eine größere Menge an Zellaggregaten unter denselben Bedingungen gebildet werden können und beispielsweise für vergleichende Experimente verwendet werden können oder statistisch bessere Ergebnisse liefern können.

Das Substrat kann beim Kippen aus einer Anordnung, in der der Boden der Kammer im Wesentlichen horizontal angeordnet ist, um einen Winkel von 20° bis 45°, insbesondere 30° bis 40°, insbesondere 35°, gekippt werden.

Das Substrat kann insbesondere mindestens so weit gekippt werden, dass die Zellen sich an einer Seitenwand der Kammer sammeln. Das Substrat kann beispielsweise maximal so weit gekippt werden, dass die Zellen nicht aus der Kammer gelangen können, insbesondere in einen in die Kammer mündenden Fluidkanal. Insbesondere kann das Substrat so weit gekippt werden, dass sich die Zellen in einem Bereich der Kammer sammeln, also wo die Seitenwand und der Boden aufeinandertreffen.

Das Halten des Substrats kann ein Halten für mehrere Stunden, insbesondere 4 bis 26, insbesondere 6 bis 25, insbesondere 8 bis 24, insbesondere 10 bis 23, insbesondere 12 bis 22, insbesondere 14 bis 21, insbesondere 16 bis 20, insbesondere 17 bis 19, insbesondere 18 Stunden, sein. Wie oben gesehen kann die Dauer des Haltens eine empirisch oder semi-empirisch bestimmte Dauer für den jeweiligen Anwendungsfall sein oder mindestens eine Minimaldauer sein, bei der unabhängig vom Anwendungsfall Zellaggregate gebildet werden.

Während des Haltens können Umgebungsparameter, beispielsweise Umgebungstemperatur und/oder Luftfeuchtigkeit, überwacht und gegebenenfalls angepasst, insbesondere gesteuert bzw. geregelt werden. Insbesondere können dadurch für die Ausbildung des Zellaggregats geeignete Bedingungen geschaffen werden.

Das Verfahren kann, wie oben gesehen, eine Perfusion des Zellaggregats umfassen.

Die Perfusion kann umfassen, dass mittels einer oder mehrerer Pumpen Flüssigkeit in die Kammer transportiert und/oder aus dieser abtransportiert wird. Insbesondere kann Flüssigkeit durch einen Fluidanschluss und einen Fluidkanal zur Kammer hin und durch einen weiteren Fluidkanal und einen weiteren Fluidanschluss aus dieser hinaus transportiert werden.

Alternativ oder zusätzlich kann die Perfusion umfassen, dass passiv, beispielsweise mittels Gravitation, Flüssigkeit in die Kammer transportiert und/oder aus dieser abtransportiert wird. Auch dabei kann Flüssigkeit insbesondere durch einen Fluidanschluss und einen Fluidkanal zur Kammer hin und durch einen weiteren Fluidkanal und einen weiteren Fluidanschluss aus dieser hinaus transportiert werden.

Jeder der Fluidanschlüsse kann an der Oberseite des Substrats angeordnet sein.

Passive Systeme bieten den Vorteil, dass sie keine geschlossenen Strukturen mit Schläuchen erfordern, sondern teilweise offen sein können. Außerdem kann die Perfusion im einfachsten Fall durch Kippen oder Schwenken erfolgen.

Bei der Pumpe kann es sich um eine druckgetriebene oder peristaltische Pumpe handeln. Insbesondere kann auch ein Pumpensystem umfassend mehrere Pumpen verwendet werden.

Ein passiver Flüssigkeitstransport mittels Gravitation kann durch Kippen des Substrats oder durch unterschiedlich hoch gefüllte Reservoire bei den Fluidanschlüssen erfolgen.

Insbesondere wenn die Perfusion umfasst, dass passiv Flüssigkeit in die Kammer transportiert und/oder aus dieser abtransportiert wird, kann das Substrat zum Transport der Flüssigkeit gekippt werden. Die Flüssigkeit wird dann also bewegt, indem das Substrat gekippt wird. Beispielsweise kann das Substrat um seine Querachse gekippt werden, um Flüssigkeit entlang seiner Längsachse zu transportieren.

Die Perfusion kann umfassen, dass das Zellaggregat im Flüssigkeitsstrom angeordnet ist oder, dass das Zellaggregat superperfundiert wird. Wenn das Zellaggregat im Flüssigkeitsstrom angeordnet ist, wird es durch die Flüssigkeit umspült. Beim Superperfundieren strömt die Flüssigkeit oberhalb des Zellaggregats an diesem vorbei, beispielsweise am oberen Rand der Kammer. Wie die Perfusion erfolgt, kann insbesondere durch die Anordnung der Fluidkanäle im Substrat, durch die die Flüssigkeit in die Kammer transportiert und aus dieser heraus transportiert wird, insbesondere die Anordnung ihrer Mündung in die Kammer eingestellt werden.

Ein Substrat kann genau für eines der Perfusionsverfahren ausgebildet sein, beispielsweise, wenn genau zwei Fluidkanäle vorgesehen sind. Münden beide Fluidkanäle oben in die Kammer, ist das Substrat für Superperfusion ausgebildet, mündet einer davon oben und einer unten in die Kammer oder münden beide unten in die Kammer, kann das Zellaggregat umströmt werden, aber eine Superperfusion ist nicht möglich. Wenn mindestsens drei Fluidkanäle, insbesondere mit eigenem Fluidanschluss, in die Kammer münden, wobei beispielsweise zwei Mündungen oben und eine Mündung unten vorgesehen ist, kann durch Verwendung zweier ausgewählter Fluidkanäle bei der Perfusion zwischen den beiden Möglichkeiten gewählt werden.

Das Verfahren kann eine mikroskopische Untersuchung des Zellaggregats bzw. der Zellaggregate in der Kammer, insbesondere durch den Boden des Substrats, umfassen.

Das Zellaggregat kann vor, während und/oder nach der Perfusion in der Kammer beobachtet werden. Insbesondere kann eine solche Untersuchung erfolgen, wenn das Substrat horizontal angeordnet ist. Insbesondere wenn das Substrat und die Kammer geeignete Eigenschaften aufweisen, beispielsweise, wenn eines der unten in diesem Zusammenhang beschriebenen Substrate verwendet wird, kann hochauflösende Mikroskopie durchgeführt werden. Die mikroskopische Untersuchung kann eine Untersuchung des Wachstums umfassen. Das Verfahren kann alternativ oder zusätzlich umfassen, dass eine Fluoreszenzuntersuchung zur Bestimmung der Vitalität von Zellen durchgeführt wird.

Insbesondere können unter Verwendung der Zellaggregate in dem Substrat Wirkstofftests durchgeführt werden und dabei insbesondere eine mikroskopische Untersuchung durchgeführt werden. Wenn ein Substrat mit mehreren Kammern verwendet wird, können insbesondere unabhängige Tests gleichzeitig durchgeführt werden.

Wenn in einem Substrat mehrere Kammern ausgebildet sind, kann das Verfahren umfassen, bei der Perfusion durch jede der Kammern separat eine Flüssigkeit zu transportieren, insbesondere zu zirkulieren. Das Verfahren kann also umfassen, dass mehrere Zellaggregate unabhängig voneinander jeweils mit einer eigenen Flüssigkeit perfundiert werden. Insbesondere können so auch Wirkstofftests durchgeführt werden, wobei insbesondere mehrere Zellaggregate gleichzeitig untersucht werden und für verschiedene Zellaggregate unterschiedliche Wirkstoffkonzentrationen verwendet werden können und/oder eines oder mehrere der Zellaggregate als Referenzproben untersucht werden können.

Das Substrat kann oberhalb der bzw. jeder Kammer eine Öffnung aufweisen, die zumindest während der Bildung des Zellaggregats und gegebenenfalls der Perfusion, insbesondere mit einem Deckel, beispielsweise einer Folie, verschlossen ist. Das Verfahren kann eine Entnahme der Zellen, insbesondere des bzw. der Zellaggregate, durch die Öffnung aus dem Substrat umfassen. Bei der Öffnung handelt es sich insbesondere um eine Öffnung unmittelbar nach außen. Zur Entnahme wird der Deckel abgenommen und anschließend werden die Zellen, insbesondere das bzw. die Zellaggregate, durch die Öffnung unmittelbar aus der jeweiligen Kammer entnommen. Das Substrat kann in diesem Fall mehrteilig ausgebildet sein und ein Unterteil, in dem der Boden und die Seitenwand bzw. Seitenwände ausgebildet sind, und den Deckel umfassen, wobei der Deckel lösbar an dem Unterteil befestigt ist und im befestigten Zustand die Öffnung verschließt und zumindest einen Teil der Decke der Kammer bildet.

Der Vorteil ist, dass so ein direkter Zugriff auf das Zellaggregat möglich ist, so dass es, beispielsweise für weitere Untersuchungen, optional auch im Ganzen, entnommen werden kann.

Die Erfindung betrifft auch ein System umfassend ein Substrat, in dem eine Kammer mit mindestens einer Seitenwand, einem Boden, und einer Decke ausgebildet ist, eine Kippvorrichtung, an der das Substrat angeordnet, insbesondere befestigt, ist und die zum Kippen des Substrats in eine gekippte Ausrichtung und Halten des Substrats in der gekippten Ausrichtung ausgebildet ist, und eine Steuereinrichtung, die derart ausgebildet ist, dass sie die Kippvorrichtung so steuert, dass sie das Substrat in die gekippte Ausrichtung kippt und das Substrat in der gekippten Ausrichtung hält. Das Substrat kann insbesondere an einer Drehachse der Kippvorrichtung befestigt sein. Die Steuereinrichtung kann insbesondere ausgebildet sein, die Kippeinrichtung so zu steuert, dass die Kippeinrichtung das Substrat so kippt, dass, wenn sich in der Kammer Zellen befinden, die Zellen sich an einer Seitenwand der Kammer ansammeln. Die Steuereinrichtung kann alternativ oder zusätzlich ausgebildet sein, die Kippeinrichtung so zu steuert, dass die das Substrat so in der gekippten Ausrichtung hält, dass, wenn sich in der Kammer Zellen befinden, die Zellen sich an einer Seitenwand der Kammer ansammeln und dort dreidimensionale Zellaggregate bilden. Insbesondere kann die Steuereinrichtung derart steuern, dass sie das Substrat für eine vorgegebene Zeit, die insbesondere derart vorgegeben ist, dass sich in Anwesenheit von Zellen in dieser Zeit ein Zellaggregat bildet, hält.

Die Kippvorrichtung kann optional zusätzlich zum Zurückkippen des Substrats ausgebildet sein, insbesondere zum Zurückkippen in eine Ausgangsposition, aus der das Substrat in die gekippte Ausrichtung gekippt wurde. Optional kann die Kippvorrichtung auch zum Kippen des Substrats durch die Ausgangsposition hindurch und dann in die Ausgangsposition ausgebildet sein. Die Steuereinrichtung kann die Kippvorrichtung derart ansteuern, dass sie das Substrat zurückkippt, insbesondere in die Ausgangsposition. Insbesondere kann die Steuereinrichtung die Kippvorrichtung derart steuern, dass sie das Substrat automatisch nach einer vorgegebenen Zeit zurückkippt, die insbesondere groß genug ist, dass sich in dieser Zeit in Anwesenheit von Zellen Zellaggregate bilden

Die Kippvorrichtung kann beispielsweise einen Antrieb, insbesondere einen Motor umfassen. Die Steuereinrichtung kann zum Steuern des Antriebs derart, dass er das Kippen und/oder Zurückkippen des Substrats antreibt, ausgebildet sein.

Das System kann auch eine oder mehrere Pumpen zum Transportieren von Flüssigkeit in die Kammer hinein und aus der Kammer heraus umfassen. Die Steuereinrichtung kann dann optional auch zum Steuern der Pumpen ausgebildet sein, derart, dass die Pumpen die Flüssigkeit entsprechend transportieren.

Das System kann zum Durchführen eines der oben beschriebenen Verfahren ausgebildet sein. Alternativ oder zusätzlich kann das System eines der im Folgenden beschriebenen Substrate umfassen.

Die Erfindung betrifft auch ein Substrat, in dem eine Kammer mit mindestens einer Seitenwand, einem, insbesondere planen, Boden, und einer Decke ausgebildet ist, wobei eine Seitenwand bzw. mindestens eine der Seitenwände und der Boden der Kammer eine zellabweisende Eigenschaft haben, insbesondere eine zellabweisende Beschichtung aufweisen.

Die Kammer kann die zellabweisende Eigenschaft in einem Bereich aufweisen, der einen Teil des Bodens und einen daran angrenzenden Teil der Seitenwand oder einen Teil des Bodens und die Kante zwischen zwei daran angrenzende Seitenwände umfasst und der, wenn der Boden aus einer horizontalen Anordnung heraus um eine in der horizontalen Ebene angeordnete Achse, insbesondere eine Längsachse, eine Querachse oder eine Diagonale, des Substrats gekippt ist, unterhalb der übrigen Bereiche der Kammer angeordnet ist. Die Seitenwand kann in dem Bereich eine Kreisbogenform oder eine Ellipsenbogenform aufweisen bzw. die Seitenwände der Kammer können in diesem Bereich eine, insbesondere abgerundete, Kante einschließen. Der Bereich kann seitlich versetzt zu der Längsachse des Substrats angeordnet sein. Alternativ oder zusätzlich kann der Grundriss der Kammer asymmetrisch sein.

Aufgrund der zellabweisenden Eigenschaft können die Zellen bei der Bildung des Zellaggregats nicht an dem Boden bzw. der Seitenwand festwachsen.

Die Größe der Kammer kann bezogen auf das Volumen mindestens die zweifache bis dreifache Größe des zu bildenden Zellaggregats haben. Insbesondere können eines, mehrere oder alle der Länge, Breite und Höhe der Kammer jeweils mindestens zwei bis drei Mal so groß sein wie die Länge bzw. Breite bzw. Höhe des zu bildenden Zellaggregats oder jeweils mindestens zwei bis drei Mal so groß wie der Durchmesser des Zellaggregats. Die Abmessungen des zu bildenden Zellaggregats hängen von der Zellzahl, dem Zelltyp und der zu bildenden Form, beispielsweise basierend auf der Form der Seitenwand bzw. Seitenwände in dem Bereich, wo die Zellaggregate gebildet werden sollen, ab. Insbesondere können basierend auf diesen Parametern die Abmessungen des zu bildenden Zellaggregats vorhergesagt werden und eine Kammer mit einer der oben beschriebenen relativen Größen verwendet werden. Die Kammer kann beispielsweise eine Höhe und Breite von jeweils 1 bis 5 mm und eine Höhe von 1 bis 10 mm, insbesondere 1 bis 8 mm, insbesondere 1 bis 4 mm, insbesondere 1 bis 3 mm, insbesondere 1 bis 2,5 mm aufweisen.

Die Kammer kann insbesondere einen Bereich aufweisen, in dem genau eine, beispielsweise kreisbogenförmige oder ellipsenbogenförmige, Seitenwand angeordnet ist oder in dem zwei Seitenwände rechtwinklig oder in einem spitzen Winkel aufeinandertreffen, wobei die Kante zwischen den beiden Seitenwänden insbesondere abgerundet sein kann. Im einem der oben beschriebenen Verfahren oder Systeme kann die kreisbogenförmige Seitenwand die Seitenwand sein, wo sich die Zellen ansammeln.

Die Kammer kann mehrere Bereiche aufweisen, die zum Ansammeln der Zellen verwendet werden können. Die Bereiche können dieselbe Form haben. Alternativ kann der Grundriss der Kammer asymmetrisch sein. Insbesondere können die Bereiche unterschiedliche Form haben. Wenn der Grundriss der Kammer asymmetrisch ist bzw. die Bereiche unterschiedliche Formen haben, können bei der Verwendung des Substrats je nach Kipprichtung Zellaggregate verschiedener Geometrien hergestellt werden.

Wenn sich die Zellen an einer geraden Begrenzung ansammeln, beispielsweise einer geraden Seitenwand, bilden sie ein stäbchenförmiges Zellaggregat mit im Wesentlichen rechteckiger Grundfläche aus. Wenn sich die Zellen an einer kreisbogenförmigen Seitenwand sammeln, ist das entstehende Aggregat stäbchenförmig mit halbkreisförmiger Grundfläche. Wenn sich die Zellen an einer abgerundeten Kante sammeln, nimmt das Zellaggregat Kugelform oder Halbkugelform an.

Die Form des Zellaggregats hat dabei Einfluss auf die Zahl der darin befindlichen lebenden Zellen. Bei einem kugelförmigen Zellaggregat, welches auch als Sphäroid bezeichnet wird, besteht bei einem Durchmesser von 400 - 500 µm der Kern in der Regel aus abgestorbenen Zellen, da nicht mehr genug Nährstoffe dorthin diffundieren können. Bei einem stäbchenförmigen Zellaggregat ist das Oberfläche-zu-Volumen-Verhältnis in der Regel derart, dass dort der Anteil der lebenden Zellen größer ist. Dies kann insbesondere für anschließende Untersuchungen außerhalb des Substrats von Vorteil sein.

Das Substrat kann ganz oder teilweise, insbesondere im Bereich des Bodens der Kammer, aus einem Material mit Brechungsindex > 1,2 und < 1,7 ausgebildet sein.

Das Substrat kann aus einem, insbesondere biokompatiblen, Kunststoff bestehen. Das Kunststoffmaterial kann beispielsweise Polycarbonat, Polystyrol, Polyethylen, Polyvinylchlorid, Cyclo-olefin Copolymer, Cyclo-olefin Polymer oder Polymethylmethacrylat umfassen. Der Kunststoff kann ein Elastomer umfassen. Das Elastomer kann ein Silikon, insbesondere Polydimethylsiloxan, PDMS, umfassen.

Das Substrat kann, insbesondere der Bereich unterhalb des Bodens der Kammer, aus Material bestehen, das derart ausgebildet ist, dass eine mikroskopische Untersuchung des Zellwachstums möglich ist. Insbesondere kann die Doppelbrechung des Materials so gering sein, dass die mikroskopische Untersuchung möglich ist und/oder die Autofluoreszenz des Materials kann so gering sein, dass eine Untersuchung mit Fluoreszenzmikroskopie möglich ist. Die Eigenfluoreszenz, kann insbesondere kleiner oder gleich der Eigenfluoreszenz von COC (Cyclo-Olefin-Copolymer) oder COP (Cyclo-Olefin-Polymer) oder eines herkömmlichen Deckglases ist. Insbesondere kann die Eigenfluoreszenz kleiner oder gleich der Eigenfluoreszenz eines herkömmlichen Deckglases, beispielsweise reinweißes Glas der hydrolytischen Klasse 1 wie Menzel-Deckglas, insbesondere mit der Stärke Nr. 1,5, sein.

Mit einem derart optisch hochwertigen Material lassen sich in vorteilhafter Weise Mikroskopie-untersuchungen durchführen. Beispielsweise kann die Doppelbrechung so gering sein, dass DIC (Differential Interference Contrast) möglich ist. Eine geringe Eigenfluoreszenz erlaubt die Durchführung von Fluoreszenzmessungen.

Das Substrat kann in Form eines Mikroskopieträgers ausgebildet sein.

Das Substrat kann einen ersten und einen zweiten Fluidkanal umfassen, die, insbesondere an gegenüberliegenden Enden der Kammer, in die Kammer münden und die jeweils mit einem separaten Fluidanschluss verbunden sind. Dabei können beide Fluidkanäle in den oberen Bereich der Kammer münden oder beide Fluidkanäle in den unteren Bereich der Kammer münden oder einer der Fluidkanäle in den oberen Bereich der Kammer und der andere Fluidkanal in den unteren Bereich der Kammer münden. Jeder der Fluidanschlüsse kann an der Oberseite des Substrats angeordnet sein.

Das Substrat kann zusätzlich zu dem ersten und dem zweiten Fluidkanal mindestens einen dritten Fluidkanal umfassen, der in die Kammer mündet und mit einem separaten Fluidanschluss verbunden ist. Jeder der Fluidanschlüsse kann beispielsweise an der Oberseite des Substrats angeordnet sein. Einer der Fluidkanäle kann in den unteren Bereich der Kammer münden und die beiden anderen Fluidkanäle können an in den oberen Bereich der Kammer münden, wobei insbesondere einer der im oberen Bereich in die Kammer mündenden Fluidkanäle an einem Ende in die Kammer mündet, das dem Ende gegenüberliegt, in der der im unteren Bereich in die Kammer mündende Fluidkanal in die Kammer mündet. Gegenüberliegenden. Wie oben erläutert, kann so je nach Verwendung der Fluidanschlüsse eine Umströmung oder eine Superperfusion erfolgen

Eine der Seitenwände bzw. ein Abschnitt der Seitenwand der Kammer kann mit dem Boden der Kammer einen stumpfen Winkel einschließen, insbesondere die Seitenwand bzw. der Abschnitt der Seitenwand, die bzw. der zwischen dem Boden der Kammer und der Mündung eines oben in die Kammer mündenden Fluidkanals angeordnet ist. Die Seitenwand bzw. der Abschnitt können also schräg bzw. geneigt sein. So können ungünstige Strömungsverhältnisse, beispielsweise Staupunkte, an den Kanten und Ecken der Kammer vermieden werden.

Die Kammer kann einen planen Boden aufweisen, der insbesondere im Wesentlichen parallel zu der Unterseite des Substrats sein kann. Zusätzlich kann auch die Oberseite des Substrats im Wesentlichen parallel zur Unterseite des Substrats und dem Boden sein. Die Oberseite und/oder Unterseite des Substrats können ebenfalls plan sein. Ein Substrat mit einem planen Boden ist für eine Untersuchung mit hochauflösender Mikroskopie geeignet. Bei unebenen Böden, beispielsweise bei den oft für die Assemblierung verwendeten Wellplatten mit rundem Boden, ist dies aufgrund optischer Verzerrungen nicht möglich.

Weitere Merkmale und Vorteile werden nachfolgend anhand der beispielhaften Figuren erläutert. Dabei zeigen:
- Figur 1a bis 1c: schematische, nicht-maßstabsgetreue Seitenansicht eines Substrats und Draufsichten auf ein Substrat einer ersten Ausführungsform mit zwei alternativen Grundrissen der Kammer,
- Figur 2: eine schematische, nicht-maßstabsgetreue Seitenansicht eines Substrats einer zweiten Ausführungsform,
- Figur 3: eine schematische, nicht-maßstabsgetreue Seitenansicht eines Substrats einer dritten Ausführungsform,
- Figur 4: eine schematische, nicht-maßstabsgetreue Seitenansicht eines Substrats einer vierten Ausführungsform,
- Figur 5: eine schematische, nicht-maßstabsgetreue Seitenansicht eines Substrats einer fünften Ausführungsform,
- Figuren 6a und 6b: schematische, nicht-maßstabsgetreue Draufsicht auf ein Substrat und Seitenansicht eines Substrats einer sechsten Ausführungsform mit einer Mehrzahl von Kammern,
- Figuren 7a und 7b: schematische, nicht-maßstabsgetreue Draufsicht auf ein Substrat und Seitenansicht eines Substrats einer siebten Ausführungsform mit einer Mehrzahl von Kammern,
- Figuren 8a bis 8f: schematische, nicht-maßstabsgetreue Darstellungen verschiedener Grundrisse der Kammer,
- Figur 9: eine schematische, nicht-maßstabsgetreue Darstellung eines Systems einer Ausführungsform, und
- Figuren 10a bis 10h: schematische Darstellung verschiedener möglicher Schritte eines Verfahrens einer Ausführungsform.

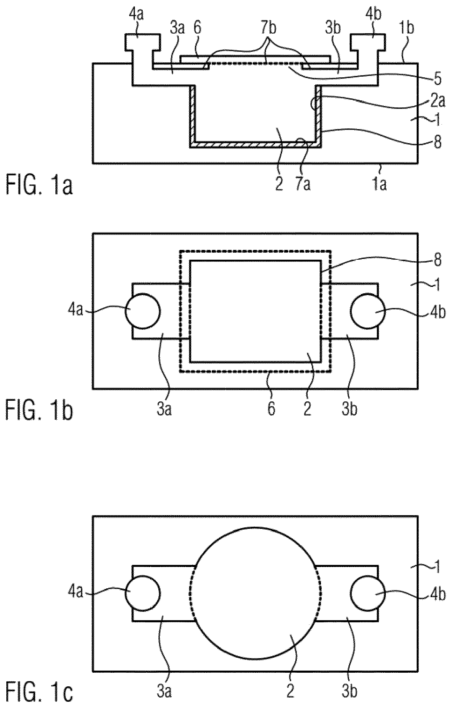

In Figur 1a ist eine Seitenansicht eines Substrats 1 mit eine Kammer 2, zwei Fluidkanälen 3a und 3b, und zwei Fluidanschlüssen 4a und 4b. Ein Ende der Fluidkanäle mündet in die Kammer und das andere Ende in den jeweiligen Fluidanschluss. Mindestens einer der Fluidanschlüsse kann ein Flüssigkeitsreservoir umfassen oder ein Flüssigkeitsreservoir kann auf den Fluidanschluss aufgesetzt sein.

In Figur 1a ist beispielhaft ein Substrat gezeigt, das oberhalb der Kammer eine Öffnung 5 aufweist, die während der Bildung des Zellaggregats mit einem abnehmbaren Deckel 6, beispielsweise einer Folie, verschlossen ist. Eine solche Öffnung ist jedoch optional.

Das Substrat kann, wenn es eine solche Öffnung aufweist, mehrteilig ausgebildet sein und ein Unterteil, in dem der Boden und die Seitenwand bzw. Seitenwände der Kammer ausgebildet sind, und den Deckel umfassen, wobei der Deckel lösbar an dem Unterteil befestigt ist und im befestigten Zustand die Öffnung verschließt und zumindest einen Teil der Decke der Kammer bildet.

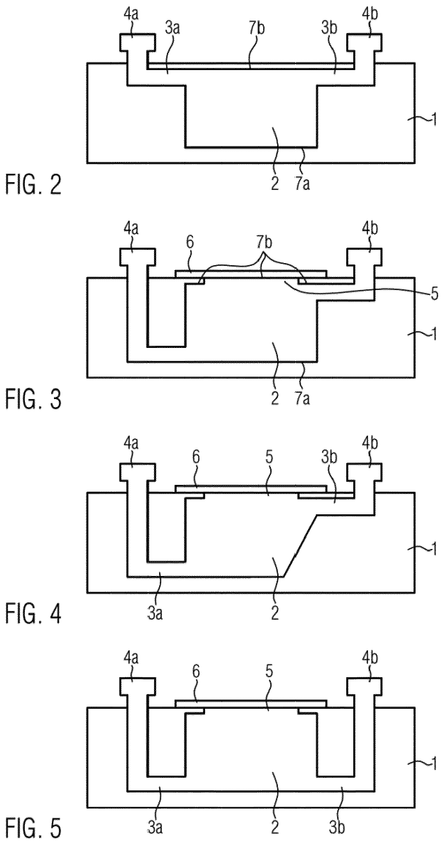

Das Substrat kann alternativ beispielsweise derart ausgebildet sein, dass keine Öffnungen unmittelbar oberhalb der Kammer ausgebildet sind. Das Substrat kann insbesondere derart ausgebildet sein, dass es nur mit Fluidanschlüssen direkt verbundene Öffnungen aufweist. Ein Substrat ohne die Öffnung 5 ist beispielhaft in Figur 2 dargestellt, wobei das Substrat ansonsten so ausgebildet ist, wie im Zusammenhang mit Figur 1 beschrieben.

Die Kammer hat einen Boden 7a, der insbesondere plan ausgebildet und parallel zur Unterseite 1a, die in diesem Beispiel ebenfalls plan ausgebildet ist, und optional, wie in diesem Beispiel, auch parallel zur Oberseite 1b des Substrats angeordnet ist. Die Oberseite kann ebenfalls plan ausgebildet sein, gegebenenfalls abgesehen von den Fluidanschlüssen und der Öffnung. Insbesondere kann der Deckel 6 plan sein.

Die Kammer kann verschiedene Grundrisse aufweisen, beispielsweise rechteckig oder rund. In Figur 1b ist beispielhaft eine Draufsicht auf das Substrat mit einem rechteckigen Grundriss und in Figur 1c eine Draufsicht mit rundem Grundriss gezeigt.

Wenn das Substrat einen runden oder ellipsenförmigen Grundriss hat, hat es eine umlaufende Seitenwand 8, die gegebenenfalls durch Mündungen von Fluidkanälen unterbrochen sein kann. Wenn die Seitenwand einen rechteckigen Grundriss hat, weist sie vier Seitenwände 8a, 8b, 8c, 8d auf, die ebenfalls gegebenenfalls durch Mündungen von Fluidkanälen unterbrochen sein können. Die Seitenwand 8 oder eine oder mehrere der Seitenwände 8a bis 8d, insbesondere alle Seitenwände können im Wesentlichen senkrecht zum Boden der Kammer angeordnet sein. Dies ist jedoch optional, wie unten noch im Detail erläutert.

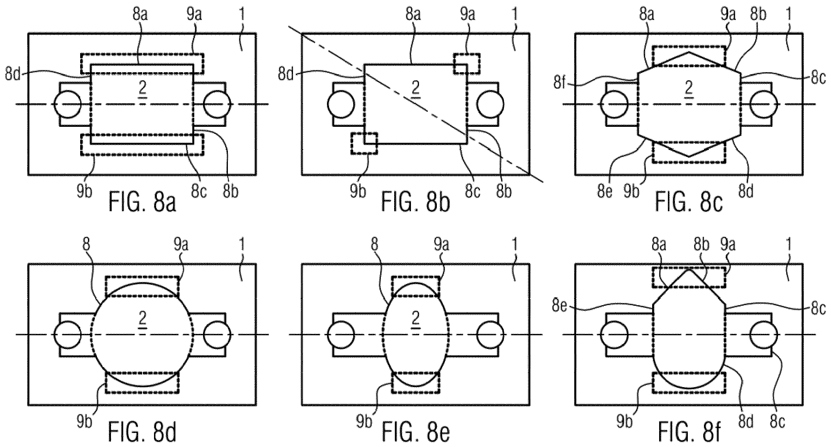

Insbesondere kann sie jeden der unten im Zusammenhang mit Figuren 8a bis 8f beschriebenen Grundrisse aufweisen. Dort sind auch mit einer gestrichelten Linie Bereiche 9a und 9b der Kammer gekennzeichnet, an denen sich jeweils durch Kippen des Substrats in eine bestimmte Richtung und um einen vorgegebenen Winkel Zellen 16, die sich in der Kammer befinden, ansammeln und ein Zellaggregat 10 bilden. Die Drehachse und Richtung, in die das Substrat dazu gekippt wird, sind dabei jeweils eine Drehachse und Richtung, die dazu führt, dass durch das Kippen der entsprechende Bereich der Kammer relativ zum Rest der Kammer weiter unten angeordnet wird. Beispiele, wie diese Bereiche der Kammer ausgebildet sein können, sind unten anhand der Figuren 8a bis 8f erläutert, wobei jede der dort gezeigten Formen der Kammer mit der in Figur 1a gezeigten grundsätzlichen Ausgestaltung des Substrats kombiniert werden können.

Die Wand bzw. Wände und der Boden der Kammer können zumindest in dem bzw. den Bereichen eine zellabweisende Eigenschaft haben, insbesondere eine zellabweisende Beschichtung 2a aufweisen. Insbesondere kann der gesamte Boden und/oder die gesamte Seitenwand bzw. die gesamten Seitenwände, eine zellabweisende Eigenschaft aufweisen.

Das Substrat kann insbesondere in Form eines Mikroskopieträgers ausgebildet sein, insbesondere, zumindest oberhalb und/oder unterhalb der Kammer, aus einem im allgemeinen Teil beschriebenen optisch hochwertigen Material.

In dem in Figuren 1a bis 1c und 2 gezeigten Beispielen münden beide Fluidkanäle in den oberen Bereich der Kammer. In Figur 2 ist das Substrat beispielhaft ohne eine Öffnung über den Fluidkanal dargestellt. Ein solches Substrat kann insbesondere einteilig ausgebildet sein.

In Figur 3 ist ein Substrat gezeigt, das so wie die Substrate in Figuren 1 und 2 ausgebildet sein kann (also mit oder ohne Öffnung oberhalb der Kammer), mit dem Unterschied, dass in dem Substrat in Figur 3 der Fluidkanal 3a in den unteren Bereich der Kammer mündet und der Fluidkanal 3b in den oberen Bereich der Kammer mündet. Auch in diesem Beispiel kann die Kammer jeden der unten im Zusammenhang mit Figuren 8a bis 8f beschriebenen Grundrisse aufweisen.

Optional kann noch ein dritter Fluidkanal vorgesehen sein, der oberhalb des Fluidkanals 3b, insbesondere seitlich versetzt zu diesem, verläuft und in den oberen Bereich der Kammer mündet. Der dritte Fluidkanal kann mit einem eigenen Fluidanschluss verbunden sein. So kann mit demselben Substrat wahlweise eine Umströmung oder eine Superperfusion erfolgen.

In Figur 4 ist ein Substrat gezeigt, das so wie die Substrate in Figuren 1 und 2 ausgebildet sein kann (also mit oder ohne Öffnung oberhalb der Kammer), mit dem Unterschied, dass in dem Substrat in Figur 4 der Fluidkanal 3a in den unteren Bereich der Kammer mündet und der Fluidkanal 3b in den oberen Bereich der Kammer mündet. In diesem Beispiel ist eine der Seitenwände bzw. ein Abschnitt der Seitenwand der Kammer gegenüber dem Boden der Kammer geneigt, insbesondere so, dass die Seitenwand und der Boden einen stumpfen Winkel einschließen. Hier ist insbesondere die Seitenwand bzw. der Abschnitt der Seitenwand geneigt angeordnet, die zwischen dem Boden der Kammer und der Mündung des oben in die Kammer mündenden Fluidkanals 3b angeordnet ist. Auch in diesem Beispiel kann die Kammer jeden der unten im Zusammenhang mit Figuren 8a bis 8f beschriebenen Grundrisse aufweisen, gegebenenfalls mit der Einschränkung, dass der Bereich, wo die Zellaggregate gebildet werden, nicht dort angeordnet ist, wo einer der Fluidkanäle unten in die Kammer mündet.

In Figur 5 ist ein Substrat gezeigt, das so wie die Substrate in Figuren 1 und 2 ausgebildet sein kann (also mit oder ohne Öffnung oberhalb der Kammer), mit dem Unterschied, dass in dem Substrat in Figur 5 beide Fluidkanäle 3a und 3b in den unteren Bereich der Kammer münden.

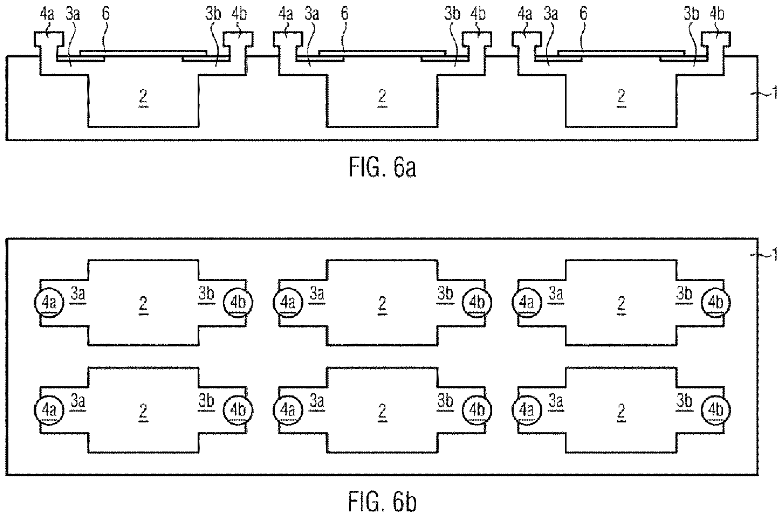

In Figuren 6a und 6b sind eine Draufsicht und eine Seitenansicht eines Substrats mit einer Mehrzahl von Kammern 2 gezeigt. Dabei können alle Kammern gleich geformt sein oder sie können verschiedene Formen aufweisen. Jede der Kammern kann beispielsweise so ausgebildet sein, wie oben im Zusammenhang mit Figuren 1 bis 5 beschrieben. Jede Kammer ist mit zwei eigenen Fluidanschlüssen 4a und 4b verbunden.

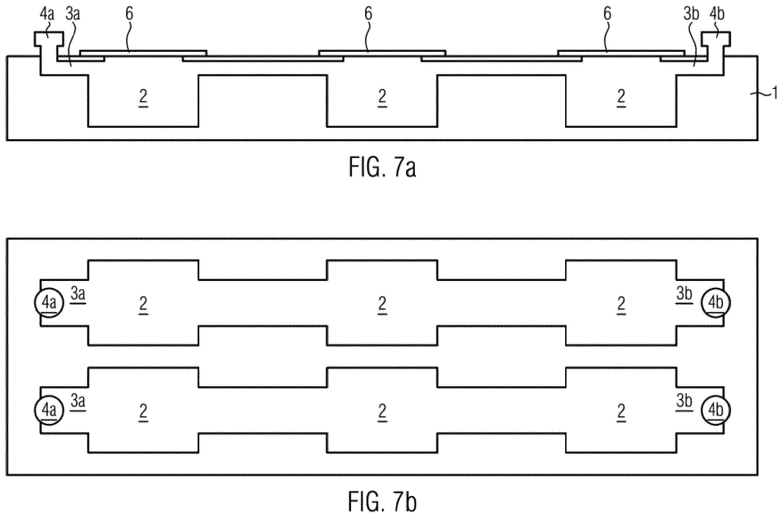

Alternativ kann auch eine Gruppe von mehreren Kammern jeweils über einen Fluidkanal mit gemeinsamen Fluidanschlüssen verbunden sein. Das Substrat kann genau eine oder mehrere solcher Gruppen aufweisen. Ein Beispiel mit zwei solchen Gruppen ist in Figuren 7a und 7b beispielhaft in Seitenansicht und Draufsicht gezeigt.

In den Figuren 6a, 6b, 7a und 7b sind beispielhaft sechs Kammern in dem Substrat dargestellt. Diese Anzahl kann aber beliebig abgewandelt werden. Insbesondere können beispielsweise zwölf oder 24 Kammern in dem Substrat vorgesehen sein und gegebenenfalls beliebig gruppiert sein.

In Figuren 8a bis 8f sind verschiedene mögliche Grundrisse einer Kammer gezeigt, die beispielsweise für jedes der oben beschriebenen Substrate verwendet werden können. Beispielhafte Anordnungen der Fluidkanäle sind gestrichelt dargestellt. Beispielhafte Kippachsen und die sich durch Kippen um diese Achse ergebenden Bereiche 9a und 9b, in denen sich Zellen ansammeln sind in den Figuren gestrichelt gekennzeichnet.

In Figur 8a ist eine Kammer mit rechteckigem Grundriss gezeigt. In Figur 8b ist ebenfalls eine Kammer mit rechteckigem Grundriss gezeigt, wobei hier die Kante zwischen den Fluidkanalwänden 8a und 8b abgerundet ist. In Figur 8c ist eine Kammer mit polygonem Grundriss, hier mit sechs Kanten, gezeigt. In Figur 8d ist eine Kammer mit rundem Grundriss gezeigt. In Figur 8e ist eine Kammer mit elliptischen Grundriss gezeigt.

In Figur 8f ist eine Kammer gezeigt, die einen ersten Bereich 9a aufweist, bei dem die Seitenwände 8a und 8b senkrecht aufeinandertreffen, wobei die Kante, wie hier gezeigt, optional abgerundet sein kann. Weiterhin weist die Kammer einen zweiten Bereich 9b auf, welcher dem ersten Bereich gegenüberliegt. In diesem Bereich ist die Seitenwand in Form eines Kreisbogens oder Ellipsenbogens ausgebildet. Die anderen Seitenwände sind in diesem Beispiel eben. Im vorliegenden Beispiel sind die ebenen Seitenwände die, in die die Fluidkanäle münden.

Insbesondere im Falle eines nicht-rechteckigen und nicht-kreisförmigen Grundrisses und/oder eines asymmetrischen Grundrisses, ist ein großes Maß an Flexibilität hinsichtlich der Geometrie der sich bildenden Zellaggregate möglich. Abhängig davon, in welche Richtung das Substrat gekippt wird, können Zellaggregate mit verschiedenen Geometrien erzeugt werden.

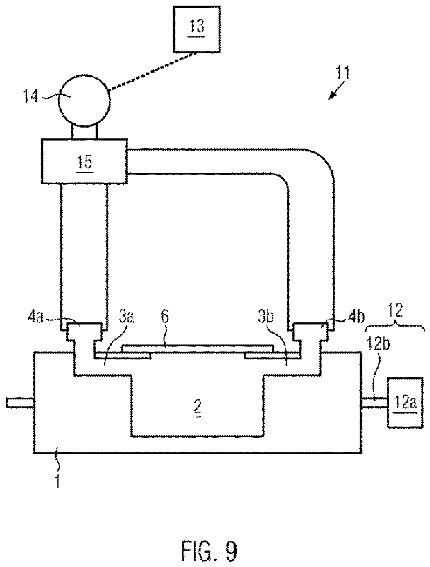

Figur 9 zeigt ein System 11 mit einem Substrat, wobei hier beispielhaft das Substrat aus Figur 1 gezeigt ist und eine Kippvorrichtung 12, die derart ausgebildet ist, dass sie das Substrat kippt, insbesondere automatisch, und in einer gekippten Position hält. Die Kippvorrichtung kann auch zum Zurückkippen des Substrats, insbesondere automatisch, ausgebildet sein. Beispielsweise kann die Kippvorrichtung einen Motor 12a umfassen, der das Substrat kippt, und eine Achse 12b, an der das Substrat befestigt ist und die vom Motor rotiert wird, so dass das Substrat gekippt wird. Das System umfasst eine Steuereinrichtung 13, die hier beispielsweise den Motor steuert. Die Kippvorrichtung 12 kann zum Kippen des Substrats um eine oder mehrere Achsen ausgebildet sein.

Das System kann optional auch eine oder mehrere Pumpen 14 umfassen, die derart mit dem Substrat verbunden sind, dass sie Flüssigkeit aus einem Behälter 15 durch die Fluidanschlüsse, Fluidkanäle und gegebenenfalls auch die Kammer pumpen können. Die Pumpen können mittels einer Steuereinrichtung, insbesondere mittels der Steuereinrichtung 13 gesteuert werden. Die Pumpen sind nicht zwangsläufig vorgesehen. Ein Flüssigkeitstransport kann alternativ auch durch Kippen des Substrats erfolgen, wie unten im Zusammenhang mit dem Verfahren erläutert.

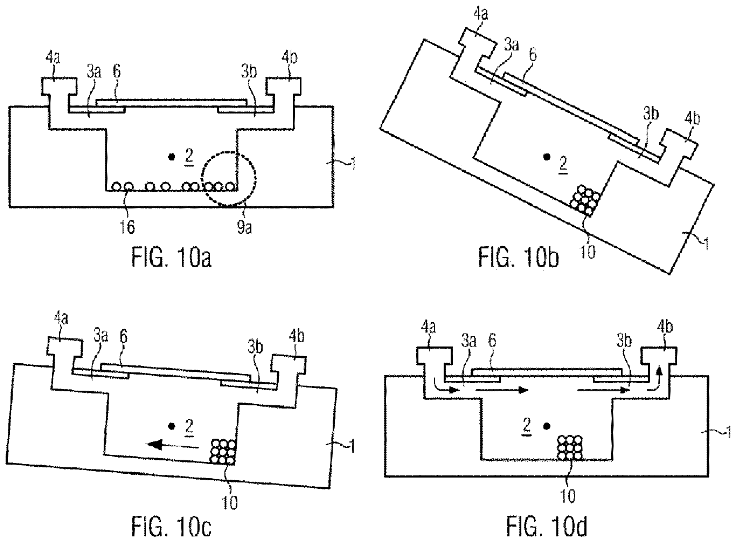

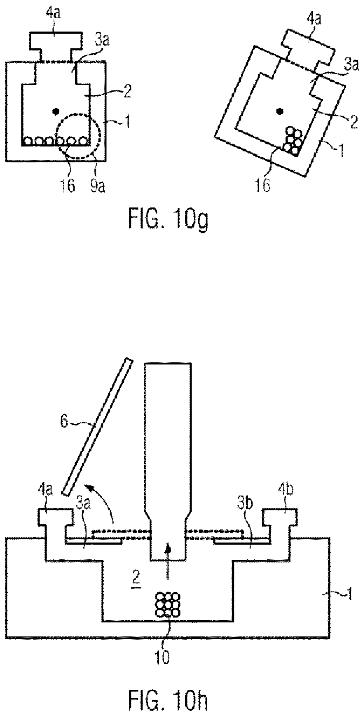

Im Folgenden wird anhand der Figuren 10a bis 10g ein Verfahren zur Kultivierung von Zellen in einem dreidimensionalen Zellaggregat in einem Substrat 1, in dem eine Kammer ausgebildet ist, 2 erläutert. Es kann das beispielsweise unter Verwendung eines der oben beschriebenen Substrate und gegebenenfalls Systeme durchgeführt werden. Auch andere geeignete Substrate bzw. Systeme können verwendet werden.

Das Verfahren umfasst, dass Zellen 16 in die Kammer 2 eingebracht werden. Dabei ist im vorliegenden Beispiel das Substrat derart angeordnet, dass der Boden der Kammer im Wesentlichen horizontal ist. Danach sind die Zellen etwa gleichmäßig, insbesondere in einer Monolage, in der Kammer verteilt, wie in Figur 10a gezeigt.

Anschließend wird das Substrat derart in eine gekippte Ausrichtung gekippt, dass die Zellen sich an einer Seitenwand der Kammer ansammeln. Beispielsweise kann das Substrat um 20° bis 45° gekippt werden. Dieser Zustand ist in Figur 10b gezeigt. In Figur 10b ist das Substrat um die Querachse gekippt, die hier senkrecht zur Zeichenebene ist.

Im nächsten Schritt wird das Substrat über einen vorgegebenen Zeitraum, beispielsweise von mehreren Stunden, insbesondere zwischen 17 und 19 Stunden, insbesondere 18 Stunden, in der gekippten Ausrichtung gehalten. Die Zellen und das Substrat sind derart ausgebildet, dass sich in diesem Zeitraum Zellaggregate 10 bilden. Insbesondere ist dabei die Form des Bereichs der Kammer, wo sich die Zellen ansammeln, für die Form der Zellaggregate maßgeblich. Die zellabweisende Eigenschaft der Seitenwand bzw. Seitenwände und des Bodens der Kammer in diesem Bereich ermöglichen, , dass die Zellen dort nicht anwachsen. Die Seitenwand bzw. Seitenwände bilden zusammen mit dem Boden eine Begrenzung für das Zellaggregat und beeinflussen somit die Form, in der das Zellaggregat wächst. Unter anderem aufgrund der Gravitation und/oder Oberflächenspannung, sowie Wachstumseigenschaften der Zellen, ist es nicht erforderlich, das Zellaggregat von allen Seiten zu begrenzen, um die gewünschte Form zu erhalten.

Optional wird das Substrat anschließend zurückgekippt. Aufgrund der Gravitation und gegebenenfalls der Oberflächenbeschaffenheit der Kammer bewegt sich das Zellaggregat 10 von der Seitenwand bzw. den Seitenwänden weg Richtung Mitte der Kammer, wie in Figur 10c gezeigt. Optional kann das Substrat durch die Ausgangsposition hindurch weiter gekippt werden und danach wieder zurück in die Ausgangsposition gekippt werden (hier nicht gezeigt).

In der Kammer kann eine Perfusion des Zellaggregats durchgeführt werden. Dazu wird Flüssigkeit, beispielsweise Zellkulturmedium, durch einen ersten Fluidanschluss 4a und einen ersten Fluidkanal 3a in das Substrat eingeleitet und durch einen zweiten Fluidkanal 3b und einen zweiten Fluidanschluss 4b aus dem Substrat ausgeleitet.

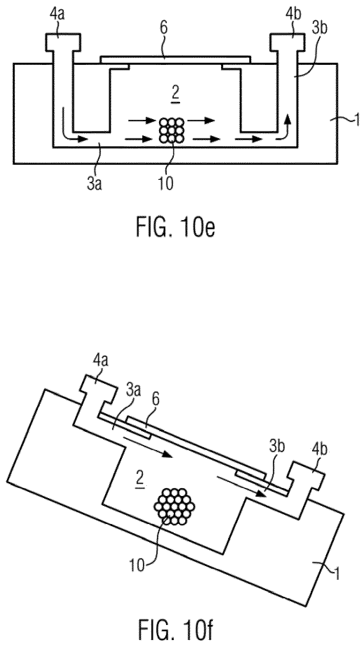

Beim Durchströmen des Substrats kann die Flüssigkeit nur am Rand der Kammer entlang strömen, wie in Figur 10d gezeigt. Das ist beispielswiese der Fall, wenn die beiden Fluidkanäle im oberen Bereich der Kammer in diese münden. In diesem Fall erfolgt eine Superperfusion, das heißt, die Flüssigkeit umströmt das Zellaggregat nicht, sondern strömt oberhalb daran vorbei. Alternativ kann die Flüssigkeit das Zellaggregat umströmen, wie beispielsweise in Figur 10e gezeigt, beispielsweise, wenn einer oder beide Fluidkanäle unten in die Kammer münden.

Die Perfusion kann im Anschluss an das Zurückkippen erfolgen. Alternativ kann die Perfusion bereits während des Zurückkippens erfolgen.

Das Verfahren ist oben anhand eines Substrats, in dem eine Kammer ausgebildet ist, beschrieben. Wenn ein Substrat mit mehreren Kammern verwendet wird, beispielsweise wie in Figuren 6a, 6b, 7a, 7b gezeigt, können auch mehrere Zellaggregate gleichzeitig gebildet werden, indem in mehrere Kammern Zellen eingebracht werden.

Auch die Perfusion kann dann für mehrere Zellaggregate, insbesondere gleichzeitig, erfolgen. Die Perfusion der einzelnen Zellaggregate kann für alle Zellaggregate unabhängig voneinander erfolgen, wenn jede Kammer mit zwei eigenen Fluidanschlüssen verbunden ist, beispielsweise wie in Figuren 6a und 6b gezeigt. Dazu wird für jede der Kammern jeweils über einen der beiden Fluidanschlüsse Flüssigkeit in die Kammer transportiert.

Alternativ kann auch einer Gruppe mit mehreren Kammern über einen geteilten Fluidanschluss Flüssigkeit zugeführt werden, beispielweise bei einem wie in Figuren 7a und 7b gezeigten Substrat. So kann eine einheitliche Perfusion der Zellaggregate innerhalb einer Gruppe erfolgen. Das Substrat kann mehrere solche Gruppen aufweisen. Dann kann für Zellaggregate verschiedener Gruppen beispielsweise eine Perfusion mit Flüssigkeit in einer anderen Zusammensetzung erfolgen.

Insbesondere bei Verwendung eines Substrats mit einer Vielzahl von Kammern, wie beispielsweise in Figuren 6a, 6b, 7a und 7b gezeigt, können so auch parallele unabhängige Wirkstoffmessungen durchgeführt werden.

Die Perfusion kann mittels einer oder mehrerer Pumpen erfolgen, wie beispielsweise in einem System wie in Figur 9. Alternativ kann die Perfusion aufgrund von Gravitation durch Kippen des Substrats erfolgen, wie in Figur 10f gezeigt. Beispielsweise kann das Substrat abwechselnd in entgegengesetzte Richtung gekippt werden, um eine Strömung zu erhalten.

In Figur 10b wird das Substrat um die Querachse gekippt. Alternativ kann es auch um die Längsachse gekippt werden. Insbesondere, wenn die Bereiche, die für die Bildung der Zellaggregate in Querrichtung versetzt zur Längsachse sind. Alternativ oder zusätzlich kann das Substrat auch um andere Achsen, beispielsweise eine der Diagonalen gekippt werden.

Das Verfahren kann optional umfassen, dass eine mikroskopische Untersuchung des Zellaggregats bzw. der Zellaggregate in der Kammer durchgeführt wird, insbesondere durch den Boden des Substrats. Insbesondere kann ein hochauflösendes Mikroskopie-Verfahren durchgeführt werden, vor allem, wenn der Boden der Kammer plan ist. Diese Untersuchung erfolgt vorzugsweise, wenn das Substrat horizontal ausgerichtet ist. Sie kann vor, während und/oder nach der Perfusion erfolgen.

Das Verfahren kann ebenfalls optional umfassen, dass Zellen, insbesondere das bzw. die Zellaggregate, aus dem Substrat entnommen werden, insbesondere nach der Perfusion und gegebenenfalls einer mikroskopischen Untersuchung. Wenn die Zellaggregate nicht im Ganzen entnommen werden, können die Zellen optional lysiert und die resultierende Zellsuspension entnommen werden, beispielsweise abgesaugt werden. Wenn das Substrat, wie in Figur 1 gezeigt, eine mit einem Deckel verschlossene Öffnung aufweist, beispielsweise ein Deckel in Form einer Folie, beispielsweise Klebefolie, kann zur Entnahme der Deckel abgenommen werden, so dass Zellen unmittelbar aus der Kammer entnommen werden können. In dem Fall kann optional das Zellaggregat als Ganzes, beispielsweise mit einer Pipette, entnommen werden. Dies ist schematisch in Figur 10h gezeigt.

Es versteht sich, dass in den zuvor beschriebenen Ausführungsbeispielen genannte Merkmale nicht auf diese speziellen Kombinationen beschränkt sind und auch in beliebigen anderen Kombinationen möglich sind.

## Patentansprüche

1. Verfahren zur Kultivierung von Zellen in einem Substrat (1), in dem eine Kammer (2) mit mindestens einer Seitenwand (8, 8a, 8b, 8c, 8d, 8e, 8f), einem, insbesondere planen, Boden (7a), und einer Decke (7b) ausgebildet ist, umfassend
Einbringen von Zellen (16) in die Kammer (2);
Kippen des Substrats (1) in eine gekippte Ausrichtung derart, dass die Zellen (16) sich an einer Seitenwand (8, 8a, 8b, 8c, 8d, 8e, 8f) der Kammer (2) ansammeln; und
Halten des Substrats (1) in der gekippten Ausrichtung, so dass die Zellen (16) ein dreidimensionales Zellaggregat (10) bilden.

2. Verfahren nach Anspruch 1, wobei das Substrat (1) mehrere Kammern (2) umfasst und vor dem Kippen in jede der Kammern (2) Zellen (16) eingebracht werden, so dass durch das Kippen und Halten des Substrats (1) gleichzeitig in jeder der Kammern (2) ein Zellaggregat (10) gebildet wird, wobei das Verfahren insbesondere eine Perfusion eines oder mehrere der, insbesondere aller, Zellaggregate (10), insbesondere unabhängig voneinander, umfasst.

3. Verfahren nach Anspruch 1 oder 2, wobei das Substrat (1) beim Kippen aus einer Anordnung, in der der Boden (7a) der Kammer (2) im Wesentlichen horizontal angeordnet ist, um einen Winkel von 20° bis 45°, insbesondere 30° bis 40°, insbesondere 35°, gekippt wird.

4. Verfahren nach einem der vorangegangenen Ansprüche, wobei das Halten des Substrats (1) ein Halten für mehrere Stunden, 4 bis 26 Stunden, insbesondere 6 bis 25 Stunden, insbesondere 8 bis 24 Stunden, insbesondere 10 bis 23 Stunden, insbesondere 12 bis 22 Stunden, insbesondere 14 bis 21 Stunden, insbesondere 16 bis 20 Stunden, insbesondere 17 bis 19 Stunden, insbesondere 18 Stunden, ist.

5. Verfahren nach einem der vorangegangenen Ansprüche, wobei das Verfahren eine Perfusion des Zellaggregats (10) in der Kammer (2), insbesondere während und/oder nach einem Zurückkippen des Substrats (1), insbesondere einem Zurückkippen in eine Ausgangsposition, aus der das Substrat (1) in die gekippte Ausrichtung gekippt wurde.

6. Verfahren nach einem der Ansprüche 2 bis 5, wobei die Perfusion umfasst, dass mittels einer oder mehrerer Pumpen (14) Flüssigkeit in die Kammer (2) transportiert und/oder aus dieser abtransportiert wird und/oder dass passiv, beispielsweise mittels Gravitation, Flüssigkeit in die Kammer (2) transportiert und/oder aus dieser abtransportiert wird.

7. Verfahren nach einem der Ansprüche 2 bis 6, wobei die Perfusion umfasst, dass passiv Flüssigkeit in die Kammer (2) transportiert und/oder aus dieser abtransportiert wird, wobei zum Transport der Flüssigkeit das Substrat (1) gekippt wird.

8. Verfahren nach einem der Ansprüche 2 bis 6, wobei die Perfusion umfasst, dass das Zellaggregat (10) im Flüssigkeitsstrom angeordnet ist oder dass das Zellaggregat (10) superperfundiert wird.

9. Verfahren nach einem der vorangegangenen Ansprüche, umfassend eine mikroskopische Untersuchung des Zellaggregats (10) bzw. der Zellaggregate (10) in der Kammer (2), insbesondere durch die Unterseite (1a) des Substrats (1).

10. Verfahren nach einem der vorangegangenen Ansprüche, wobei das Substrat (1) oberhalb der bzw. jeder Kammer (2) eine Öffnung aufweist, die während der Bildung des Zellaggregats (10) und gegebenenfalls der Perfusion mit einem Deckel (6), beispielsweise einer Folie, verschlossen ist, und
wobei das Verfahren eine Entnahme der Zellen (16), insbesondere des bzw. der Zellaggregate (10), aus dem Substrat (1) durch die Öffnung umfasst, wobei zur Entnahme der Deckel (6) abgenommen und anschließend die Zellen (16), insbesondere das bzw. die Zellaggregate (10), durch die Öffnung unmittelbar aus der jeweiligen Kammer (2) entnommen werden.

11. System umfassend
ein Substrat (1), in dem eine Kammer (2) mit mindestens einer Seitenwand (8, 8a, 8b, 8c, 8d, 8e, 8f), einem Boden (7a), und einer Decke (7b) ausgebildet ist,
eine Kippvorrichtung (12), an der das Substrat (2) angeordnet, insbesondere befestigt, ist und die zum Kippen des Substrats (1) in eine gekippte Ausrichtung und Halten des Substrats (1) in der gekippten Ausrichtung ausgebildet ist, und
eine Steuereinrichtung (13), die derart ausgebildet ist, dass sie die Kippvorrichtung (12) derart steuert, dass die Kippvorrichtung (12) das Substrat (1) in die gekippte Ausrichtung kippt und das Substrat (1) in der gekippten Ausrichtung hält.

12. Substrat (1), in dem eine Kammer (2) mit mindestens einer Seitenwand (8, 8a, 8b, 8c, 8d, 8e, 8f), einem planen Boden (7a), und einer Decke (7b) ausgebildet ist,
wobei eine Seitenwand bzw. eine der Seitenwände (8, 8a, 8b, 8c, 8d, 8e, 8f) und der Boden (7a) der Kammer (2) eine zellabweisende Eigenschaft aufweisen, insbesondere eine zellabweisende Beschichtung (2a) aufweisen,
wobei Seitenwand bzw. eine der Seitenwände (8, 8a, 8b, 8c, 8d, 8e, 8f) und der Boden (7a) der Kammer (2) die zellabweisende Eigenschaft in einem Bereich (9a, 9b) aufweisen, der einen Teil des Bodens (7a) und einen Teil der daran angrenzenden Seitenwand (8, 8a, 8b, 8c, 8d, 8e, 8f) oder eine Kante zwischen zwei der daran angrenzenden Seitenwände (8, 8a, 8b, 8c, 8d, 8e, 8f) umfasst und der, wenn der Boden (7a) aus einer horizontalen Anordnung heraus um eine in der horizontalen Ebene angeordnete Achse, insbesondere eine Längsachse, eine Querachse oder eine Diagonale, des Substrats gekippt ist, unterhalb der übrigen Bereiche der Kammer (2) angeordnet ist, und
wobei die Seitenwand in dem Bereich (9a, 9b) eine Kreisbogenform oder eine Ellipsenbogenform aufweist bzw. wobei die Seitenwände (8, 8a, 8b, 8c, 8d, 8e, 8f) der Kammer in diesem Bereich eine, insbesondere abgerundete, Kante einschließen.

13. Substrat (1) nach Anspruch 12, wobei der Bereich seitlich versetzt zu der Längsachse des Substrats angeordnet ist.

14. Substrat (1) nach Anspruch 12 oder 13, wobei der Grundriss der Kammer (2) asymmetrisch ist.
